# EUROPÄISCHE PATENTSCHRIFT

(11) **EP 3 413 846 B1**
(45) Veröffentlichungstag und Bekanntmachung des Hinweises auf die Patenterteilung: **07.10.2026**
(21) Anmeldenummer: 17708685.7
(22) Anmeldetag: 09.02.2017
(51) Int. Cl.: A61F 2/97, A61F 2/966

(54) **FREISETZUNGSSYSTEM FÜR EINE SELBST EXPANDIERENDE ENDOPROTHESE**
RELEASE SYSTEM FOR A SELF-EXPANDING ENDOPROSTHESIS
SYSTÈME DE LIBÉRATION D'UNE ENDOPROTHÈSE AUTO-EXPANSIBLE

(30) Priorität: 09.02.2016 DE 102016102212
(43) Veröffentlichungstag der Anmeldung: 19.12.2018
(73) Patentinhaber: Bentley InnoMed GmbH, 72379 Hechingen (DE)
(72) Erfinder: OBRADOVIC, Milisav, 79539 Lörrach (DE); OBRADOVIC, Aleksandar, 79539 Lörrach (DE)
(74) Vertreter: Hoefer & Partner Patentanwälte mbB
(86) Internationale Anmeldenummer: PCT/EP2017/052810
(87) Internationale Veröffentlichungsnummer: WO 2017/137471

(56) Entgegenhaltungen:
- EP-A1- 2 749 258
- US-A- 5 824 041
- US-A1- 2004 143 315
- US-A1- 2006 015 171
- US-A1- 2010 049 297
- US-B1- 8 641 752

## Beschreibung

Die Erfindung betrifft ein Freisetzungssystem für eine selbst expandierende Endoprothese mit einem Katheter, einer Endoprothese, die unter einem äußeren Zwang eine erste volumenreduzierte Form aufweist und nach Wegfall des äußeren Zwangs eine zweite expandierte Form am Ort des Einsatzes annimmt und beweglich im Katheter angeordnet ist, ein Mittel zum Halten der Endoprothese im Katheter sowie einem Mittel, das geeignet ist, den äußeren Zwang auf die Endoprothese auszuüben. Die Endoprothese ist insbesondere ein vaskulärer Stent.

Endoprothesen, insbesondere Stents für das vaskuläre System, sind in vielerlei Form bekannt. Unterschieden wird bei Stents zwischen ballonexpandierbaren Stents und selbst expandierenden Stents. Beide Varianten werden über speziell dafür konstruierte Katheter implantiert. Bei der Implantierung kommt es sehr darauf an, dass der Stent exakt platziert wird. Dies ist insbesondere bei längeren selbst expandierenden Stents, wie sie häufig peripher eingesetzt werden, ein Problem.

Selbst expandierende Stents werden aus einer Formgedächtnislegierung, beispielsweise Nickel-Titan-Legierungen (Nitinol) gefertigt und für die Platzierung im Katheter in eine erste volumenreduzierte Form gebracht, d.h. sie haben einen reduzierten Durchmesser. Im Katheter behalten sie durch den äußeren Zwang, den das Katheterrohr ausübt, diesen reduzierten Durchmesser bei. Beim Herausschieben aus dem Katheter nehmen diese Stents dann ihre zweite expandierte Form mit einem größeren Durchmesser an, den sie am Implantationsort beibehalten. Da die Expansion unmittelbar bei Verlassen des Katheters eintritt und damit diese Stents vorübergehend ein expandiertes distales Ende und ein volumenreduziertes proximalen Ende aufweisen, und bei der Freisetzung aus dem Katheter Reibungskräfte wirksam werden, leidet die Präzision der Platzierung. Hierzu trägt auch bei, dass bei der Expansion des Stents bei Austritt aus dem Katheter eine Längenreduktion stattfindet.

Um diesem Problem zu begegnen wurden verschiedene Techniken entwickelt. Eine Methode beruht auf der Anwendung einer mechanischen Platzierungshilfe, die den Stent kontrolliert aus dem Katheter hinausschiebt. Bekannt ist auch der Einschluss des Stents in eine Hülle, die am Einsatzort mit Hilfe eines im Stent angeordneten Ballons zum Platzen gebracht wird; der Stent kann expandieren, der Ballon wird mit dem Katheter aus dem Gefäßsystem zurückgezogen. Beide Methoden sind konstruktiv aufwendig. Bei letzterer Methode kann Hüllenmaterial im Gefäßsystem verbleiben. Weiterhin zeigen die Druckschriften US2004/143315A1, US2006/015171A1, US8641752B1, und US5824041A verschiedene Möglichkeiten, um einen selbstexpandierenden Stent freizusetzen. Es bleibt jedoch weiterhin das Bedürfnis nach einer verbesserten Technik zur Implantation selbstexpandierender Stents.

Der Erfindung liegt damit die Aufgabe zu Grunde, ein Freisetzungssystem für selbst expandierende Endoprothesen, insbesondere Stents, bereitzustellen, das es erlaubt, die Endoprothese am Einsatzort mit einfachen Mitteln zuverlässig und präzise freizusetzen.

Diese Aufgabe wird mit einem Freisetzungssystem gemäß Anspruch 1 gelöst. Das Mittel zur Ausübung des äußeren Zwangs auf die Endoprothese ist eine Schlauchfolie, die die Endoprothese in ihrer volumenreduzierten Form einschließt und mit ihrem proximalen Ende zum proximalen Ende des Katheters reicht, dergestalt, dass sie von der Endoprothese unter Aufhebung des äußeren Zwangs abgezogen werden kann.

Im Folgenden wird die Erfindung anhand eines selbst expandierenden Stents beschrieben, der von einer Schlauchfolie in der volumenreduzierten Form gehalten wird.

Die Schlauchfolie stellt ein wirksames Mittel zum Einschluss des Stents dar. Der Schlauch kann mit geringem Kraftaufwand abgezogen werden und damit der Stent expandiert werden. Es versteht sich, dass der Stent während des
Freisetzungsprozesses über das Haltemittel, etwa einen freigelegten Pusher, in seiner Position gehalten werden muss.

Vorzugsweise liegt die Schlauchfolie distal mit ihrer Außenseite an dem Stent an, verläuft zum distalen Ende des Stents und ist dort auf sich selbst zurückgeschlagen (umgekrempelt) um über den Stent zum proximalen Ende des Katheters zurück zu laufen.

Insbesondere setzt die Schlauchfolie am proximalen Ende des Stents an, verläuft über die gesamte Länge des Stents, um an dessen distalem Ende umzukehren und zum proximalen Katheterende zurück zu laufen. Die Schlauchfolie deckt somit den Stent über seine gesamte Länge doppelt ab. Dabei kann der äußere Schlauch, auf dem die Zugbelastung liegt, stärker und stabiler ausgeführt sein, als der auf dem Stent aufliegende Teil.

Die Schlauchfolie, wie sie erfindungsgemäß eingesetzt wird, stellt eine Hülle dar, die den Stent in seiner volumenreduzierten ersten Form einschließt und hält. Für die Freisetzung wird er vom behandelnden Arzt durch die Schleuse des Katheters hindurch soweit zurückgezogen, dass der Stent freigesetzt wird. Vorzugsweise wird gleichzeitig mit der Freisetzung des Stents auch dessen Verbindung zum Haltemittel gelöst.

Die doppelte Abdeckung des Stents durch die Schlauchfolie hat den Vorteil, dass das Abziehen mit einem sehr geringen Kraftaufwand erfolgen kann. Das für die Schlauchfolie verwandte Material, insbesondere ein medizinisch unbedenklicher Kunststoff und vorzugsweise PTFE, weist eine gute Gleitfähigkeit gegenüber sich selbst und anderen Materialien auf und damit eine geringe Friktion. Dies erleichtert das Hinausschieben des Stents aus dem Katheter und seine Freisetzung. Gerade bei peripheren Stents mit einer großen Länge ist die Friktion des Stents an der Katheterwand und die Kompression des Stents beim Hinausschieben ein häufig auftretendes Problem.

Um das Abziehen der Schlauchfolie zu erleichtern, kann sie mit Perforationen und Schlitzen ausgestattet sein, die beim Zurückziehen zum Aufreißen der Schlauchfolie führen. Diese Perforationen oder Schlitze sind zweckmäßigerweise entlang wenigstens einer Linie in Längsrichtung angeordnet. Dies vermeidet ein Abreißen der Schlauchfolie, die nicht in der Vaskulatur verbleiben soll.

Es ist ferner zweckmäßig, den Katheter im distalen Bereich, in dem der Stent transportiert wird, mit Perforationen zu versehen und eine Spülmöglichkeit vorzusehen, was die Gleitfähigkeit des umhüllten Stents im Katheter weiter erhöht. Die Spülflüssigkeit kann über die Perforationen oder Schlitze der Schlauchfolie auch zum umhüllten Stent durchdringen. Die Spülflüssigkeit übt im Zusammenhang eine erwünschte Schmierwirkung aus.

Zudem können Katheter, Schlauchfolie und/oder Stent eine hydrophile Beschichtung zur Verbesserung der Gleitfähigkeit aufweisen. Solche Beschichtungen sind an und für sich bei Kathetern bekannt.

Zur Verminderung der Reibung zwischen den Folienschichten auf dem Stent beim Abziehen kann es hilfreich sein, die äußere Folienschicht mit Perforationen zu versehen, die den Zutritt von Flüssigkeit ermöglicht. Diese Perforationen sind, im Gegensatz zu etwaigen Perforationen einer Aufreißhilfe, über die Fläche verteilt.

Das Mittel zum Halten und Verschieben des Stents kann ein Führungsdraht oder Pusher sein, der jeweils mit dem Stent gekoppelt ist oder an den Stent angrenzt. Nach der Trennung des Stents vom Katheter wird die Haltefunktion benötigt, um den Stent während des Abziehens der Folie in Position zu halten. Beispielsweise kann der Führungsdraht oder Pusher über eine geeignet gestaltete Haltevorrichtung an seinem distalen Ende von innen in die Maschen des nicht expandierten Stents eingreifen; diese Verbindung löst sich mit der Freisetzung des Stents und seiner Expansion.

Bevorzugt ist ein Führungsdraht oder Pusher, der über die Schlauchfolie mit dem Stent verbunden ist und durch Abziehen der Schlauchfolie von dem Stent getrennt wird. Dazu kann der Führungsdraht an seinem distalen Ende beispielsweise eine Scheibe aufweisen, die in ihrem Durchmesser dem Durchmesser des Stents in seiner volumenreduzierten Form entspricht und die unmittelbar an den Stent heranreicht. Sobald die Schlauchfolie vom Stent abgezogen ist, expandiert der Stent über seine gesamte Länge und ist vom Führungsdraht getrennt bzw. verliert seinen Kontakt zum angrenzenden Pusher getrennt.

Als Pusher kommt auch ein separater Schlauch infrage, der mit seinem distalen Ende an das proximale Ende des Stents reicht und geeignet ist, diesen beim Zurückziehen des Katheters in Position zu halten. Hierzu kann das proximale Ende dieses separaten Schlauches verstärkt sein, als Wulst ausgebildet sein oder umgeschlagen sein, um ein ausreichendes Widerlager für den Stent auszubilden. Der separate Schlauch läuft koaxial innerhalb der Schlauchfolie zum proximalen Ende des Katheters und kann vom behandelnden Arzt durch die Schleuse bedient werden.

Wird der Pusher von einem separaten Schlauch gebildet, hat dieser in der Regel eine stärkere Wandung, als die Schlauchfolie, damit er die zum Platzieren des Stents notwendige Schubwirkung übertragen kann.

Der separate Schlauch des Pushers kann gemäß einer weiteren Ausführungsform mit der Schlauchfolie, die den Stent einschließt, verbunden sein, beispielsweise verschweißt sein. In diesem Fall schließt er an das unmittelbar auf dem Stent liegende distale Ende der Schlauchfolie an.

Um die Reibung zwischen den beiden Schichten der Schlauchfolie, d.h. dem distalen Ende der Schlauchfolie, das unmittelbar auf den Stent angeordnet ist, und dem proximalen Teil, der vom distalen Ende des Stents zum proximalen Ende des Katheters zurückläuft und auf das distale Ende der Schlauchfolie umgeschlagen ist, gering zu halten, kann es sinnvoll sein, den proximalen Teil der Schlauchfolie im Durchmesser etwas weiter zu halten, als das distale Ende.

Es ist ferner möglich, für den über den Stentverlauf hinausgehenden Teil des proximalen Teils der Schlauchfolie ein Schlauchmaterial zu verwenden, das über eine größere Zugfestigkeit verfügt.

Andere Ablösemechanismen können ebenfalls eingesetzt werden. Beispielsweise kann eine mechanische Verbindung des Führungsdrahts zum Stent in bekannter Weise elektrolytisch gelöst werden.

Die hier verwandten Begriffe "proximal" und "distal" sind gleichbedeutend mit "dem behandelnden Arzt oder Katheterende zugewandt" bzw. "vom behandelnden Arzt oder Katheterende wegweisend".

Die Erfindung wird durch die Abbildungen bevorzugter Ausführungsformen näher erläutert. Es versteht sich, dass die darin gezeigten und nachstehen näher beschriebenen Merkmale generell auf die hier beschriebene Erfindung gelesen werden können und nicht nur auf den dargestellten Einzelfall bezogen sind.

Von den Abbildungen zeigt
- Figur 1: schematisch und im Schnitt eine 1. Variante eines erfindungsgemäßen Freisetzungssystems;
- Figur 2: eine 2. Variante eines erfindungsgemäßen Freisetzungssystems;
- Figur 3: einen Schnitt entlang der Linie A-A durch das Freisetzungssystem gemäß Figur 2;
- Figur 4: das Freisetzungssystem gemäß Figur 2 von der Stirnseite her;
- Figur 5: eine Schlauchfolie, zur Fixierung eines Stens und
- Figur 6: das erfindungsgemäße Freisetzungssystem im Prinzip.

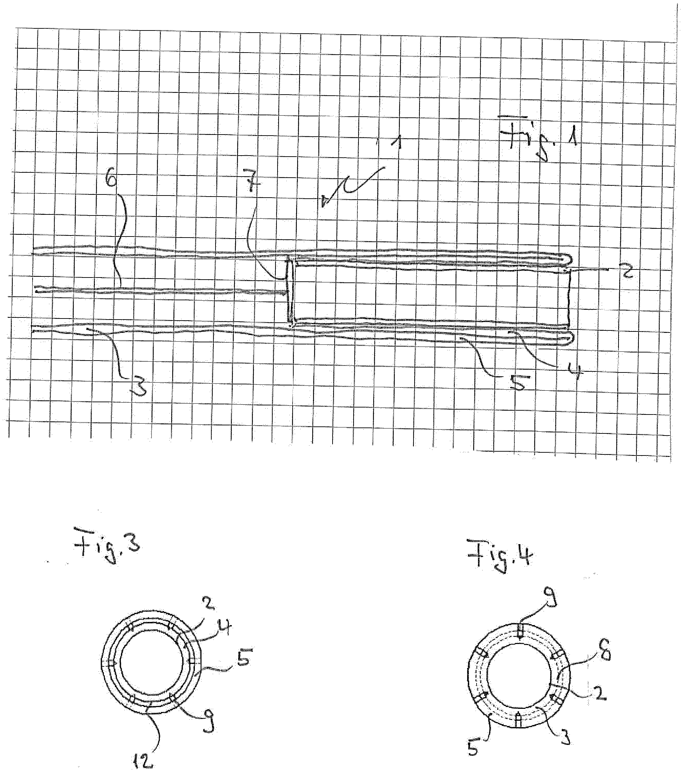

Figur 1 zeigt im Schnitt eine erste Variante eines erfindungsgemäßen Freisetzungssystems 1, bei dem ein Stent 2 innerhalb einer Schlauchfolie 3 angeordnet ist. Die Schlauchfolie 3 weist ein distales Ende 4 auf, das unmittelbar auf dem Stent 2 aufliegt und diesen in seiner volumenreduzierte Form hält. Der Stent 2 besteht aus einem Formgedächtnismaterial, vorzugsweise einer Nickel-Titan-Legierung, etwa Nitinol, das unter einem äußeren Zwang in seiner volumenreduzierten Form gehalten wird. Der äußere Zwang wird durch die Schlauchfolie 3 ausgeübt.

Die Schlauchfolie 3 ist unterteilt in den distalen Teil 4 und den proximalen Teil 5, wobei der distale Teil unmittelbar auf dem Stent aufliegt und diesen einschließt und der proximale Teil 5 auf den distalen Teil 4 umgeschlagen ist und zum proximalen Ende des Katheters (nicht dargestellt) zurückläuft. Der Katheter schließt die Kombination aus Stent 2 und Schlauchfolie 3 ein.

Um den Stent 2 vom Katheter zu befreien, befindet sich an dessen proximalem Ende ein Pusher, der im dargestellten Fall aus einem Pusherdraht 6 und einer am proximalen Ende des Stents 2 anliegenden Scheibe 7 besteht. Mithilfe des Pusherdrahtes 6 und der Scheibe 7 kann auf den Stent 2 die erforderliche Kraft ausgeübt werden, um ihn in seiner Position zu halten, wenn der Katheter zurückgezogen wird.

Die Freisetzung des Stents 2 erfolgt durch das Abziehen der Schlauchfolie 3. Dadurch, dass das proximale Ende 5 der Schlauchfolie auf das distale Ende 4 zurückgeschlagen ist, kann die Schlauchfolie 3 insgesamt leicht vom Stent 2 abgezogen werden. Das Abziehen kann durch die Einbringung von Perforationen, Schlitzen oder Schwächungszonen, die in Längsrichtung verlaufen, erleichtert werden. In diesem Fall lässt sich der Stent 2 freilegen, wie beim Schälen einer Banane.

Im dargestellten Fall sind Stent 2 und Scheibe 7 des Pushers nicht miteinander verbunden und werden nur über die Schlauchfolie 3 zusammengehalten. Sobald die Schlauchfolie 3 vollständig vom Stent 2 abgezogen ist, expandiert der Stent 2 und trennt sich von der Scheibe 7 des Pushers.

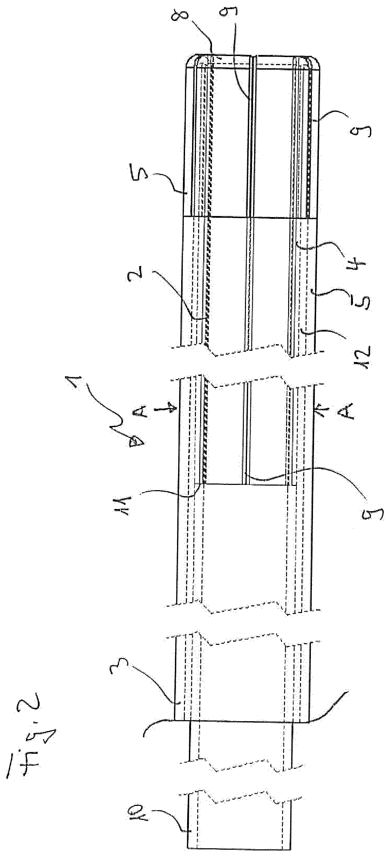

Figur 2 zeigt eine weitere Variante eines erfindungsgemäßen Freisetzungssystems 1. Der Stent 2 wird vom distalen Ende 4 der Schlauchfolie 3 in seiner volumenreduzierten Form gehalten. Wie in Figur 1 ist die Schlauchfolie 3 mit ihrem proximalen Ende 4 auf den distalen Teil 2 zurückgeschlagen. Die Bezugsziffer 8 bezeichnet den Umschlagsbereich der Schlauchfolie, die Bezugsziffer 9 auf der Schlauchfolie 3 in Bereich des Stents 2 angeordnete und längs verlaufende Schlitze, die das Aufreißen und Zurückziehen erleichtern. Generell befinden sich derartige Schwächungen der Schlauchfolie nur in dem Bereich, der den Stent 2 abdeckt.

Die Schlauchfolie 3 verläuft durch die gesamte Länge des nicht dargestellten Katheters. Sie kann vom behandelnden Arzt über ihr durch die Einführschleuse hinausragen Ende vom Stent 2 abgezogen werden.

Im dargestellten Fall besteht der Pusher aus einem Schlauch oder flexiblen Rohr 10, das innerhalb der Schlauchfolie 3 verläuft. Der Schlauch 10 ist hinreichend steif, um die zur Trennung des Stents 2 vom Katheter notwendige Kraft zu übertragen. Der Schlauch 10 ist durch Verschweißen bei 11 an das distale Ende des distalen Teils 4 der Schlauchfolie 3 angeschlossen. Alle Schläuche bzw. Schlauchteile verlaufen koaxial
Zwischen dem unmittelbar auf dem Stent 2 aufliegenden distalen Teil 4 der Schlauchfolie 3 und deren zurückgeschlagenem proximalen Teil 5 befindet sich ein Freiraum 12, der sich aus den unterschiedlichen Durchmessern der Schlauchfolienteile ergibt. Dies vermindert die Reibung und erleichtert das "Abpellen".

Figur 3 zeigt einen Querschnitt A-A durch das Freisetzungssystem gemäß Figur 2. Zu erkennen ist der schichtweise Aufbau mit dem innen angeordneten Stent 2, dem darüber angeordneten Teil 4 der Schlauchfolie, dem Zwischenraum 12 zwischen den beiden Schlauchfolienlagen und der außen nach proximal zurücklaufenden Schlauchfolie 5. Zu erkennen sind ferner Schlitze 9, die das Aufreißen der Schlauchfolie 3 beim Abziehen erleichtern.

Figur 4 zeigt in einer Stirnansicht das distale Ende der Einführvorrichtung 1 mit dem Umschlagsbereich 8 der Schlauchfolie, dem innen angeordneten Stent 2 und den Schlitzen 9, durch die das zurücklaufende proximale Ende 5 der Schlauchfolie 3 zu erkennen ist.

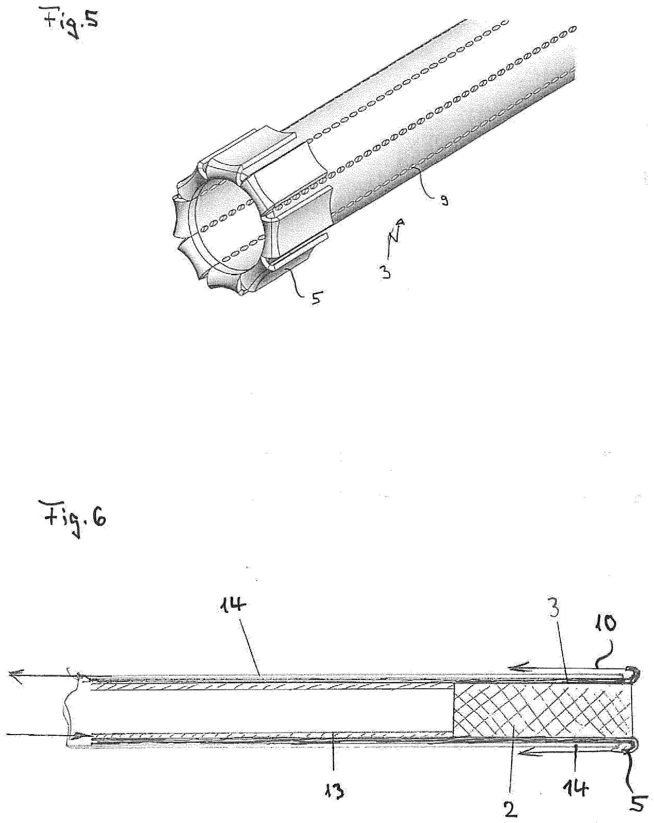

Figur 5 zeigt isoliert die Schlauchfolie 3 mit 8 über den Umfang gleichmäßig verteilten in Längsrichtung verlaufenden Perforationslinien 9, die das Aufreißen der Schlauchfolie 3 ermöglichen. Im Falle der Anwendung befindet sich die Endoprothese 2 im Inneren der Schlauchfolie 9 und der Applikationskatheter oder -schlauch auf der Außenseite. Die Schlauchfolie 3 ist an ihrem Ende umgeschlagen und bildet einzelne Zungen 5 aus. Die zwischen den Zungen 5 ausgebildeten Einschnitte verlaufen parallel zu den Perforationslinien 9, so dass bei Abziehen der Schlauchfolie mittels der Zungen 5 die Schlauchfolie entlang der Perforationslinie 9 aufreißt und die darin liegende Endoprothese, in der Regel ein selbstexpandierender Stent, freisetzt. Zum Zurückziehen der Schlauchfolie 3 ist an den Zungen beispielsweise ein Schlauch (nicht dargestellt) angeordnet. Anstelle des Abziehschlauchs können beispielsweise auch Schnüre oder Drähte verwandt werden.

Die Schlauchfolie 3 besteht vorzugsweise aus PTFE, einem Material, das wenig Reibungswiderstand liefert und damit das Zurückziehen des Schlauches erleichtert.

Figur 6 zeigt das Prinzip des erfindungsgemäßen Systems mit einem Stent 2, der darauf angeordneten Schlauchfolie 3, die am distalen Ende des Stents umgeschlagen ist und in den Zungen 5 endet, einen Pusher 13, der geeignet ist, den Stent aus dem umgebenden Katheter oder Stabilisierungsschlauch 14 herauszuschieben, sowie einen Schlauch 10, der an das umgeschlagene Ende 5 (entsprechend den Zungen 5 in Figur 5) befestigt ist, zum Abziehen der Schlauchfolie 3 vom Stent 2 nach Freisetzung des Stents 2 aus dem Katheter 14 mit Hilfe des Pushers 13. Anstelle des Schlauchs 10 können auch ein oder mehrere Zugseile verwandt werden.

In der Darstellung zeigen die Pfeile die Bewegungsrichtung der jeweiligen Elemente bei der Platzierung des Stents 2 an.

## Patentansprüche

1. Freisetzungssystem für eine selbst expandierende Endoprothese, mit einem Katheter, einer Endoprothese (2), die unter einem äußeren Zwang eine erste volumenreduzierte Form aufweist und nach Wegfall des äußeren Zwangs eine zweite expandierte Form am Ort des Einsatzes annimmt und beweglich im Katheter angeordnet ist, einem Mittel zum Halten (6,7) der Endoprothese (2) sowie einem Mittel, das geeignet ist, den äußeren Zwang auf die Endoprothese (2) auszuüben, wobei das Mittel zur Ausübung des äußeren Zwangs auf die Endoprothese (2) eine Schlauchfolie (3) ist, die mit ihrem distalen Ende (4) die Endoprothese (2) in ihrer volumenreduzierten Form einschließt und mit ihrem proximalen Ende (5) zum proximalen Ende des Katheters reicht, dergestalt, dass sie von der Endoprothese (2) unter Aufhebung des äußeren Zwangs abgezogen werden kann, wobei die Schlauchfolie (3) am proximalen Ende der Endoprothese ansetzt, über die gesamte Länge der Endoprothese zum distalen Ende der Endoprothese verläuft, dort auf sich selbst zurückgeschlagen ist und über die Endoprothese zum proximalen Ende des Katheters zurückläuft,
**dadurch gekennzeichnet dass** in der Schlauchfolie im Bereich des Stents Perforationen, Schlitze oder Schwächungszonen, die in Längsrichtung verlaufen, gleichmäßig über den Umfang verteilt angeordnet sind, wobei die Perforationen beim Zurückziehen der Schlauchfolie zum Aufreißen der Schlauchfolie führen und die Schlauchfolie einzelne Zungen ausbildet mittels derer die Schlauchfolie zurückgezogen wird und der Stent freigesetzt wird.

2. Freisetzungssystem nach Anspruch 1, **dadurch gekennzeichnet, dass** die Schlauchfolie (3) mit ihrem distalen Ende (4) am proximalen Ende der Endoprothese (2) anliegt.

3. Freisetzungssystem nach Anspruch 1 oder 2, **dadurch gekennzeichnet, dass** die Endoprothese (2) ein vaskulärer Stent ist.

4. Freisetzungssystem nach Anspruch 3, **dadurch gekennzeichnet, dass** der Stent (2) ein peripherer Stent ist.

5. Freisetzungssystem nach einem der vorstehenden Ansprüche, **dadurch gekennzeichnet, dass** die Schlauchfolie (3) aus einem medizinisch unbedenklichen Kunststoffmaterial besteht.

6. Freisetzungssystem nach Anspruch 5, **dadurch gekennzeichnet, dass** die Schlauchfolie (3) aus PTFE besteht.

7. Freisetzungssystem nach einem der vorstehenden Ansprüche, **dadurch gekennzeichnet, dass** der Katheter im distalen Bereich Perforationen aufweist.

8. Freisetzungssystem nach Anspruch 7, **dadurch gekennzeichnet, dass** der Katheter und/oder die Schlauchfolie (3) und/oder die Endoprothese (2) eine hydrophile Beschichtung aufweisen.

9. Freisetzungssystem nach Anspruch 7 oder 8, **dadurch gekennzeichnet, dass** der Katheter eine Spülvorrichtung aufweist.

10. Freisetzungssystem nach einem der vorstehenden Ansprüche, **dadurch gekennzeichnet, dass** das Mittel zum Halten (6,7) der Endoprothese (2) im Katheter ein Pusher ist, der innerhalb der Schlauchfolie (3) angeordnet ist.

11. Freisetzungssystem nach einem der Ansprüche 1 bis 9, **dadurch gekennzeichnet, dass** das Mittel zum Halten (6,7) der Endoprothese (2) im Katheter ein Pusher ist, der lösbar mit der Endoprothese (2) verbunden ist und in der Schlauchfolie (3) verläuft.

12. Freisetzungssystem nach Anspruch 11, **dadurch gekennzeichnet, dass** der Pusher (6,7) über die Schlauchfolie (3) selbstlösend mit der Endoprothese (2) verbunden ist, dergestalt, dass bei Freisetzung der Endoprothese (2) am Anwendungsort die Verbindung gelöst wird.

13. Freisetzungssystem nach Anspruch 11 oder 12, **dadurch gekennzeichnet, dass** der Pusher (6,7) ein Schlauch ist.

14. Freisetzungssystem nach einem der Ansprüche 1 bis 13, **dadurch gekennzeichnet, dass** der zurückgeschlagene proximale Teil der Schlauchfolie einen größeren Durchmesser hat als der unmittelbar auf dem Stent aufliegende distale Teil der Schlauchfolie.

15. Freisetzungssystem nach Anspruch 14, **dadurch gekennzeichnet, dass** sich zwischen dem unmittelbar auf dem Stent (2) aufliegenden distalen Teil der Schlauchfolie (3) und deren zurückgeschlagenem proximalen Teil ein Freiraum (12) befindet.

## Claims

1. Release system for a self-expanding endoprosthesis, comprising a catheter, an endoprosthesis (2) having a first volume-reduced shape under an external constraint and, after elimination of the external constraint, assuming a second expanded shape at the placement site and being movably arranged in the catheter, a means for holding (6, 7) the endoprosthesis (2) and means suitable to exert the external force on the endoprosthesis (2), wherein the means for exerting the external force on the endoprosthesis (2) is a tubular film (3) whose distal end (4) encloses the endoprosthesis (2) in its reduced-volume form and whose proximal end (5) extends to the proximal end of the catheter in such a way that it can be withdrawn from the endoprosthesis (2) thereby eliminating the external constraint, wherein the tubular film (3) is positioned at the proximal end endoprosthesis (2), extends over the whole length of the endoprosthesis to the distal end of the endoprosthesis, is folded back on itself at the distal end of the endoprosthesis and, along the endoprosthesis (2), and runs back to the proximal end of the catheter,
**characterized in that** the tubular film is provided with perforations, slots or weakening zones that extend in the longitudinal direction and are evenly distributed over the circumference, wherein the perforations lead to tearing of the tubular film when the tubular film is withdrawn, such that the tubular film forms tongues via those the tubular film is withdrawn and the stent is released.

2. Release system according to claim 1, **characterized in that** the distal end (4) of the tubular film (3) rests on the proximal end of the endoprosthesis (2).

3. Release system according to one of claims 1 or 2, **characterized in that** the endoprosthesis (2) is a vascular stent

4. Release system according to claim 3, **characterized in that** the stent (2) is a peripheral stent.

5. Release system according to any one of the preceding claims, **characterized in that** the tubular film (3) consists of a medically unobjectionable plastic material.

6. Release system according to claim 5, **characterized in that** the tubular film (3) consists of PTFE.

7. Release system according to any one of the preceding claims, **characterized in that** the catheter is provided with perforations in the distal region.

8. Release system according to claim 7, **characterized in that** the catheter and/or the tubular film (3) and/or the endoprosthesis (2) comprise a hydrophilic coating.

9. Release system according to claim 7 or 8, **characterized in that** the catheter is provided with a flushing device.

10. Release system according to any one of the preceding claims, **characterized in that** the means for holding (6, 7) the endoprosthesis (2) in the catheter is a pusher arranged within the tubular film (3).

11. Release system according to any one of claims 1 to 9, **characterized in that** the means for holding (6, 7) the endoprosthesis (2) in the catheter is a pusher connected to the endoprosthesis (2) in a detachable manner and extending within the tubular film (3).

12. Release system according to claim 11, **characterized in that** the pusher (6, 7) is connected to the endoprosthesis (2) in a self-releasing manner via the tubular film (3), in such a way that the connection becomes detached when the endoprosthesis (2) is released at the placement site.

13. Release system according to claim 11 or 12, **characterized in that** the pusher (6, 7) is a tube.

14. Release system according to any one of the preceding claims, **characterized in that** the proximal part of the tubular film that is folded back has a wider diameter than the distal end of the tubular film arranged directly on the stent.

15. Release system according to claim 14, **characterized in that** there is a clearance between distal end of the tubular film (3) arranged directly on the stent (2) and the folded back proximal part of the tubular film.

## Revendications

1. Système de libération pour une endoprothèse auto-expansible, avec un cathéter, une endoprothèse (2) qui présente une première forme réduite en volume sous contrainte extérieure et prend une seconde forme expansée au site d'insertion après suppression de la contrainte extérieure et est disposée de manière mobile dans le cathéter, un moyen de maintien (6, 7) de l'endoprothèse (2) ainsi qu'un moyen qui est adapté pour exercer la contrainte extérieure sur l'endoprothèse (2), dans lequel le moyen pour exercer la contrainte extérieure sur l'endoprothèse (2) est un film tubulaire (3) qui enferme avec son extrémité distale (4) l'endoprothèse (2) dans sa forme réduite en volume et s'étend avec son extrémité proximale (5) vers l'extrémité proximale du cathéter, de sorte qu'il puisse être retiré de l'endoprothèse (2) en supprimant la contrainte extérieure, dans lequel le film tubulaire (3) se place à l'extrémité proximale de l'endoprothèse, s'étend sur toute la longueur de l'endoprothèse jusqu'à l'extrémité distale de l'endoprothèse, où il est rabattu sur lui -même et revient à l'extrémité proximale du cathéter via l'endoprothèse,
**caractérisé en ce que** des perforations, des fentes ou des zones d'affaiblissement qui s'étendent dans la direction longitudinale sont disposées uniformément réparties sur la circonférence dans le film tubulaire dans la zone du stent, dans lequel les perforations entraînent une déchirure du film tubulaire lors du retrait du film tubulaire et le film tubulaire réalise des languettes individuelles au moyen desquelles le film tubulaire est rétracté et le stent est libéré.

2. Système de libération selon la revendication 1, **caractérisé en ce que** le film tubulaire (3) avec son extrémité distale (4) s'applique contre l'extrémité proximale de l'endoprothèse (2).

3. Système de libération selon la revendication 1 ou 2, **caractérisé en ce que** l'endoprothèse (2) est un stent vasculaire.

4. Système de libération selon la revendication 3, **caractérisé en ce que** le stent (2) est un stent périphérique.

5. Système de libération selon l'une quelconque des revendications précédentes, **caractérisé en ce que** le film tubulaire (3) est constitué d'un matériau plastique médicalement inoffensif.

6. Système de libération selon la revendication 5, **caractérisé en ce que** le film tubulaire (3) est constitué de PTFE.

7. Système de libération selon l'une quelconque des revendications précédentes, **caractérisé en ce que** le cathéter présente des perforations dans la zone distale.

8. Système de libération selon la revendication 7, **caractérisé en ce que** le cathéter et/ou le film tubulaire (3) et/ou l'endoprothèse (2) présentent un revêtement hydrophile.

9. Système de libération selon la revendication 7 ou 8, **caractérisé en ce que** le cathéter présente un dispositif de rinçage.

10. Système de libération selon l'une quelconque des revendications précédentes, **caractérisé en ce que** le moyen de maintien (6, 7) de l'endoprothèse (2) dans le cathéter est un poussoir qui est disposé à l'intérieur du film tubulaire (3).

11. Système de libération selon l'une quelconque des revendications 1 à 9, **caractérisé en ce que** le moyen de maintien (6, 7) de l'endoprothèse (2) dans le cathéter est un poussoir qui est relié de manière détachable à l'endoprothèse (2) et passe dans le film tubulaire (3).

12. Système de libération selon la revendication 11, **caractérisé en ce que** le poussoir (6, 7) est relié de manière autodétachable à l'endoprothèse (2) par l'intermédiaire du film tubulaire (3), de sorte que la liaison est rompue au site d'utilisation lors de la libération de l'endoprothèse (2).

13. Système de libération selon la revendication 11 ou 12, **caractérisé en ce que** le poussoir (6, 7) est un tube.

14. Système de libération selon l'une quelconque des revendications 1 à 13, **caractérisé en ce que** la partie proximale rabattue du film tubulaire présente un diamètre supérieur à la partie distale du film tubulaire reposant directement sur le stent.

15. Système de libération selon la revendication 14, **caractérisé en ce qu'**un espace libre (12) se trouve entre la partie distale du film tubulaire (3) reposant directement sur le stent (2) et la partie proximale rabattue de celui-ci.
